# EUROPEAN PATENT APPLICATION

(11) **EP 3 845 271 A1**
(43) Date of publication of application: **07.07.2021**
(21) Application number: 20208541.1
(22) Date of filing: 19.11.2020
(51) Int. Cl.: A61N 5/06

(54) **PHOTOTHERAPY APPARATUS PREVENTING EYE EXPOSURE AND CONTROL METHOD THEREOF**

(30) Priority: 31.12.2019 KR 20190179830
(71) Applicant: Mirint Co., Ltd, Seongnam-si, Gyeonggi-do 13449 (KR)
(72) Inventor: KIM, Yong Ho, 08845 SEOUL (KR)
(74) Representative: Groth & Co. KB

(57) **Abstract**

The present disclosure relates to a phototherapy apparatus that irradiates therapeutic light to a nasal cavity of a user. More particularly, the present disclosure relates to a phototherapy apparatus including a phototherapy apparatus body, a pair of probes inserted into a nasal cavity, a light generation unit configured to irradiate the nasal cavity with therapeutic light, at least one of the pair of probes being rotatably coupled to the phototherapy apparatus body, buttons configured to open tips of the pair of probes, a sensing unit configured to detect a manipulation of the button, and a control unit configured to control the light generation unit based on a sensing result of the sensing unit.

## Description

### BACKGROUND

The present disclosure relates to a phototherapy apparatus capable of preventing eye exposure, and more particularly, to a phototherapy apparatus in which therapeutic light is irradiated only in a state of being inserted into a nasal cavity so that the eyes of a user or people around him/her are not irradiated with therapeutic light, and a control method thereof.

As for rhinitis causing inflammation that occurs in a nasal mucosa inside the nose, which is a respiratory organ of the human body, allergic rhinitis and viral rhinitis caused by cold most commonly occur. When infected with rhinitis, the infected person not only has difficulty in breathing, but also the resulting symptoms such as a headache or decreased attention will appear.

As a phototherapy apparatus commonly used for the purpose of alleviating and treating rhinitis symptoms, a product manufactured by an ultrasonic spraying method in which therapeutic drugs, such as saline, benzenetonium chloride, alcohol, etc., are made into small particles of vapor using ultrasonic waves, and a product manufactured by a compressed air spray method in which water vapor is expanded and ejected using water vapor generated by heating the therapeutic drug or saline solution to a certain temperature are widely used.

Recently, a laser phototherapy apparatus has been developed to improve allergic rhinitis symptoms by a bio-stimulation effect of laser by irradiating the nasal cavity with a semiconductor laser by means of low-level laser therapy (LLLT).

In a process of controlling such a laser phototherapy apparatus, there is a possibility of damage to the eyes, such as when the eyes are directly irradiated with light because a power source of light is operated in a state where the laser phototherapy apparatus is not inserted into the nose.

Korean Patent Registration No. 10-1947631 operates in a way that controls to irradiate laser after a preset waiting time for safe rhinitis treatment, thereby minimizing irradiation of the eyes of a user with the laser. However, since there is a possibility that laser is irradiated even in a state where it is not actually inserted into the nasal cavity, it may be insufficient in terms of safety.

Accordingly, research on a phototherapy apparatus that irradiates a light source only in a state of being inserted into the nasal cavity and blocks the light source outside the nasal cavity is required.

### SUMMARY

The present disclosure provides a phototherapy apparatus capable of blocking therapeutic light so that therapeutic light is irradiated only inside the nasal cavity and the eyes of users or others are not irradiated with therapeutic light.

The technical problems to be achieved in the present invention are not limited to the technical problems mentioned above, and other technical problems that are not mentioned will be clearly understood by the person having ordinary skill in the art to which the present invention pertains from the following description.

In accordance with an exemplary embodiment of the present invention, a phototherapy apparatus includes a phototherapy apparatus body, a pair of probes inserted into a nasal cavity, a light generation unit configured to irradiate the nasal cavity with therapeutic light, buttons configured to open tips of the pair of probes, at least one of the pair of probes being rotatably coupled to the phototherapy apparatus body, a sensing unit configured to detect a manipulation of the button, and a control unit configured to control the light generation unit based on a sensing result of the sensing unit.

The control unit may be configured to control the light generation unit to block the therapeutic light when the manipulation is detected.

An elastic portion configured to provide restoring force in a direction in which the tips of the pair of probes are closed may be further included.

The buttons may be provided one by one on both sides of the phototherapy apparatus body, and the tips of the pair of probes may be configured to be opened as much as the buttons are pressed on both sides of the phototherapy apparatus body.

The control unit may be configured to control the light generation unit to block the therapeutic light when the tips of the pair of probes are closed by the restoring force.

The sensing unit may be configured to include a contact sensor for detecting a user's finger, and may check whether or not the buttons are manipulated through whether a contact is made to the contact senor.

In accordance with another exemplary embodiment of the present invention, a control method of a phototherapy apparatus including a pair of probes inserted into a nasal cavity, includes detecting a manipulation of buttons configured to open tips of a pair of probes and controlling a light generation unit for irradiating therapeutic light based on a detection result.

### BRIEF DESCRIPTION OF THE DRAWINGS

Exemplary embodiments can be understood in more detail from the following description taken in conjunction with the accompanying drawings, in which:
FIG. 1 is a perspective view illustrating an embodiment of a phototherapy apparatus in accordance with exemplary embodiment of the present invention;
FIG. 2 is a diagram illustrating that an angle of a laser irradiation unit illustrated in FIG. 1 is adjusted;
FIG. 3 is a block diagram illustrating a phototherapy apparatus A in accordance with an exemplary embodiment of the present invention;
FIG. 4 is a diagram for comparing relative angular changes between a pair of probes 100 according to the embodiment of the present invention before and after nasal insertion, in accordance with another exemplary embodiment of the present invention;
FIG. 5 is a diagram illustrating a detection area according to a change of a left probe 110 in accordance with another exemplary embodiment of the present invention; and
FIG. 6 is a diagram illustrating a control flow chart of the phototherapy apparatus A in accordance with still another exemplary embodiment of the present invention.

### DETAILED DESCRIPTION OF EMBODIMENTS

Hereinafter, exemplary embodiments disclosed in the present specification will be described in detail with reference to the accompanying drawings, but identical or similar elements are denoted by the same reference numerals regardless of reference numerals, and redundant descriptions thereof will be omitted. The suffixes "module" and "unit" for constituent elements used in the following description are given or used interchangeably in consideration of only the ease of preparation of the specification, and do not distinct meanings or roles by themselves. In addition, in describing the embodiments disclosed in this specification, when it is determined that a detailed description of related known technologies may obscure the subject matter of the embodiments disclosed in this specification, the detailed description thereof will be omitted. In addition, the accompanying drawings are only for making it easier to understand the embodiments disclosed in this specification, and the technical idea disclosed in this specification is not limited by the accompanying drawings, and are to be understood as including all changes, equivalents, and substitutes included in the spirit and scope of the present invention.

Terms including ordinal numbers, such as first and second, may be used to describe various constituent elements, but the constituent elements are not limited by the terms. These terms are used only for the purpose of distinguishing one constituent element from other constituent elements.

When a constituent element is referred to as being "connected" or "coupled" to another constituent element, it should be understood that the constituent element may be directly connected or coupled to the other constituent element, but other constituent elements may exist in the middle. On the other hand, when a constituent element is referred to as being "directly connected" or "directly coupled" to another constituent element, it should be understood that there is no other constituent element in the middle.

Singular expressions include plural expressions unless the context clearly indicates otherwise.

In this application, it should be understood that terms such as "include" or "have" are intended to designate the existence of features, numbers, steps, actions, constituent elements, parts, or combinations thereof described in the specification, and do not preliminarily exclude the possibility of the existence or addition of one or more other features, numbers, steps, actions, constituent elements, parts, or combinations thereof in advance.

Hereinafter, specific embodiments will be described in detail with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art.

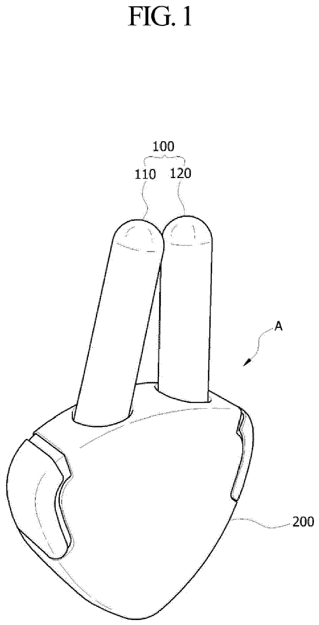

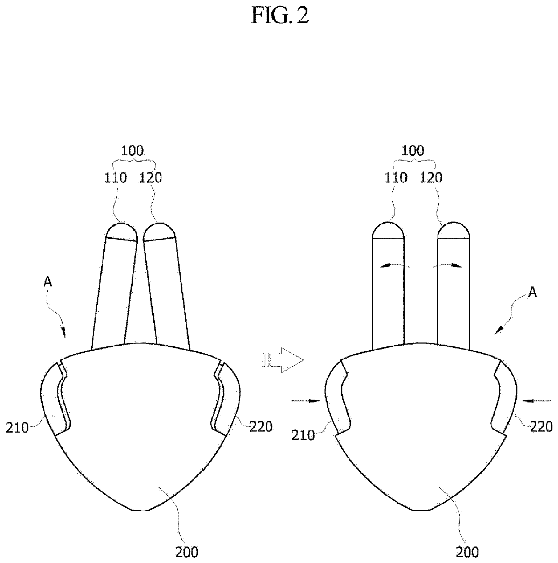

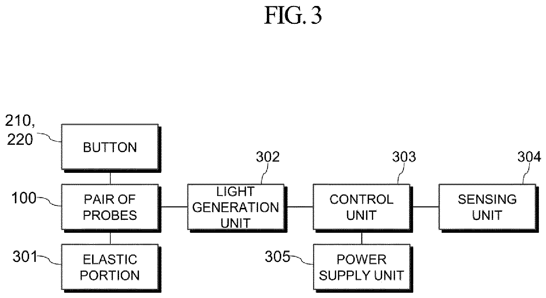

FIG. 1 is a perspective view illustrating an embodiment of a phototherapy apparatus in accordance with exemplary embodiment of the present invention, and FIG. 2 is a diagram illustrating that an angle of a laser irradiation unit illustrated in FIG. 1 is adjusted. FIG. 3 is a block diagram illustrating a phototherapy apparatus A in accordance with an exemplary embodiment of the present invention.

According to the present invention includes a pair of probes 100 inserted into the nostrils (nasal cavity) of a user to irradiate therapeutic light, and a phototherapy device body 200 in which a portion of the pair of probes 100 is embedded and at least one lower end of the pair of probes 100 is rotatable. That is, only one probe 110 or 120 of the pair of probes 100 may be pivotally coupled to the body 200, and all of the pair of probes 100 may be pivotally coupled thereto.

Referring to the block diagram of FIG. 3, the phototherapy apparatus A according to an embodiment of the present invention may be configured to include buttons 210 and 220, the pair of probes 100, an elastic portion 301, and a light generation unit 302, a control unit 303, and a sensing unit 304. The constituent elements illustrated in FIG. 3 are not essential in implementing the phototherapy apparatus A, and thus the phototherapy apparatus A described in this specification may have more or fewer constituent elements than the constituent elements listed above.

The pair of probes 100 are inserted into the nasal cavity of a user in need of treatment such as rhinitis or sinusitis, and therapeutic light generated from the light generation unit 302 may be irradiated inside the nasal cavity through the pair of probes 100.

The light generation unit 302 may be located at the tips of the pair of probes 100 to directly irradiate therapeutic light inside the nasal cavity, but the nasal cavity may be irradiated through an optical fiber provided in the pair of probes 100 in a state where the tips are located in the body 200.

The light generation unit 302 can generate therapeutic light corresponding to any one of 630 nm to 700 nm, 700 nm to 810 nm, and 810 nm to 1300 nm under the control of the control unit 303.

The control unit 303 generally controls the overall operation of the phototherapy apparatus A.

A power supply unit 305 receives external power and internal power under the control of the control unit 303 to supply power to each of the constituent elements included in the phototherapy apparatus A. The power supply unit 305 includes a battery, and the battery may be a built-in battery or a replaceable battery.

The sensing unit 304 can detect a relative angular change between the pair of probes 100 and provide the detected result to the control unit 303.

The irradiation of therapeutic light of the phototherapy apparatus (A) can be turned ON/OFF through a power button (not illustrated). On the other hand, in an embodiment of the present invention, it is proposed that the control unit 303 controls the operation of the light generation unit 302 based on the sensing result through the sensing unit 304 so that therapeutic light can be controlled in order to improve the safety of the use of the phototherapy apparatus A.

The pair of probes 100 according to an embodiment of the present invention includes a left probe 110 positioned on the left and a right probe 120 positioned on the right with the center of the body 200 illustrated in FIGS. 1 and 2 as the reference, and wavelength bands of lasers irradiated from the left and right probes 110 and 120 may be provided to be different from each other or may be provided to irradiate the same wavelength band.

Referring to FIG. 2, the pair of probes 100 are partially embedded in the body 200 as described above.

This is to enable angle adjustment with the lower end of the pair of probes 100 as the reference in order to adjust the gap between the upper ends (between the tips) of the pair of probes 100.

The elastic portion 301 that provides elastic force to the lower ends of the pair of probes 100 is provided inside the body 200 in order to adjust the angle of the pair of probes 100, and thus the angle of the pair of probes 100 can be adjusted in various ways. That is, the elastic portion 301 may provide the restoring force in a direction in which the tips of the pair of probes 100 are closed.

In addition, the phototherapy apparatus (A) according to the present invention may be provided with at least one button 210 and 220 for pressing the lower ends of the pair of probes 100 so that the lower ends of the pair of probes 100 are closed toward the center of the body 200 (i.e., opened between the tips).

That is, the buttons 210 and 220 may be provided one by one on both sides of the body 200 in order to open the tips of the pair of probes.

In normal times, the pair of probes 100 maintains a closed state between the tips by the restoring force provided by the elastic portion 301, but the pair of probes 100 may be opened between the tips when pressed by the buttons 210 and 220.

The body 200 may include various configurations related to the operation of the phototherapy device A such as driving the pair of probes 100 therein as well as the pair of probes 100 and the elastic portion 301 for adjusting the angle of the pair of probes 100.

In the corresponding configuration, for example, various semiconductor chips, microprocessors, etc. necessary for the operation of the phototherapy apparatus A may be provided, and a wireless communication module and a control module may be disposed to enable wireless interworking with a smartphone.

As described above, in the phototherapy apparatus (A) for treating diseases such as rhinitis, sinusitis, etc. by irradiating therapeutic light inside the nasal cavity, it should be avoided that the eyes are accidentally irradiated with therapeutic light. This is because if the eyes are temporarily irradiated with therapeutic light due to a manipulation mistake, it may quickly recover or it may stop at an unpleasant level, but if children play around or malfunction and the eyes are irradiated for a long time, there is a possibility of loss of vision or blindness.

Accordingly, in the present invention, it is intended to propose a method capable of irradiating therapeutic light only in a state in which a probe for delivering therapeutic light is inserted into the nasal cavity. That is, according to an embodiment of the present invention, since therapeutic light is not irradiated outside the nasal cavity, irradiation of the eyes of the user's own or others with therapeutic light is blocked.

To this end, in an embodiment of the present invention, it is proposed to detect a relative angular change between the pair of probes 100 detected through the sensing unit 304 and to control therapeutic light based on the detected result.

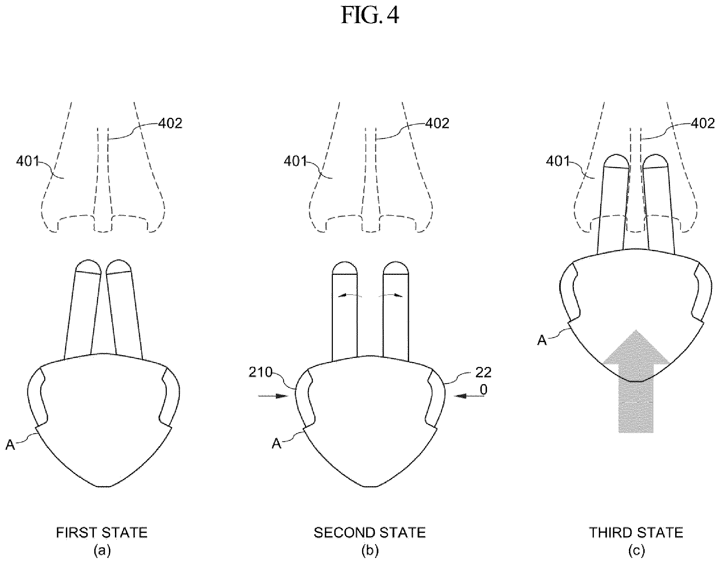

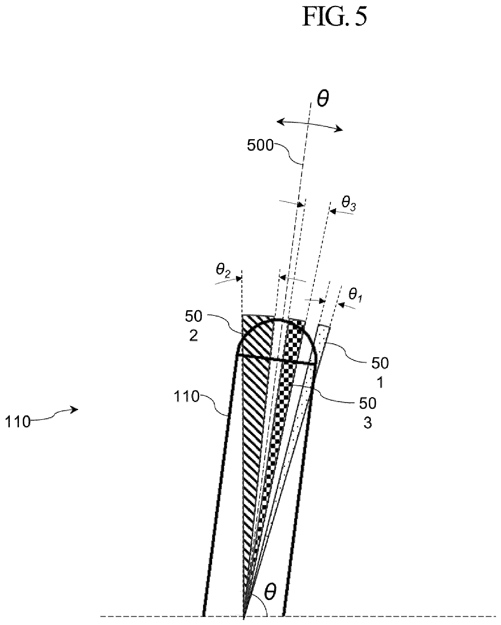

The relative angular change will be described with reference to FIGS. 4 and 5 together.

FIG. 4 is a diagram for comparing relative angular changes between the pair of probes 100 according to an embodiment of the present invention before and after nasal insertion, in accordance with another exemplary embodiment of the present invention.

First, FIG. 4A is an initial state in which no manipulation or control is applied to the phototherapy apparatus A. The pair of probes 100 is maintained in the most closed state by the elastic portion 301 described above. Hereinafter, this state of the pair of probes 100 is defined as a first state, and in this case, the tip of the pair of probes 100 is the most closed state.

When the buttons 210 and 220 are pressed so that the probe is easily inserted into a nasal cavity 401 of the user, the tip of the pair of probes 100 is opened as much as the buttons 210 and 220 are pressed. The state in which the tip is opened in this way is defined as a second state. The second state may be maintained in a state suitable for insertion into the nasal cavity 401 of the user (for example, so that the pair of probes 100 are parallel to each other).

After being inserted into the nasal cavity 401 in the second state, when the hand is released from the buttons 210 and 220, the tips of the pair of probes 100 are closed by the elastic portion 301. In this case, the pair of probes 100 may not be in the first state in which the tips are completely closed, but may be in a state in which a certain distance between the tips is maintained by a nasal septum 402. This state is defined below as a third state.

That is, the sensing unit 304 according to an embodiment of the present invention detects that a relative angle between the pair of probes 100 changes into the first state in which the hand is completely released, the second state in which the buttons 210 and 220 are pressed, and the third state which is intermediate between the first and second states. Further, based on this detection result, the control unit 303 controls irradiation/blocking of therapeutic light to irradiate therapeutic light only when the pair of probes 100 are located in the nasal cavity.

FIG. 5 is a diagram illustrating a detection area according to a change of the left probe 110 in accordance with another exemplary embodiment of the present invention

In the present invention, at least one of the left probe 110 and the right probe 120 can be rotatably coupled to the body 200, and in the illustrated example, only the left probe 110 has been described, but it will be apparent that the same can be applied to the right probe 120.

As described above in conjunction with FIG. 4, in an embodiment of the present invention, it is proposed that the entire rotational motion range of the left probe 110 is divided into a first angle range 501, a second angle range 502, and a third angle range 503 in order to detect the first to third states, and the left probe 110 is detected to be in the first to third states depending on which angle range an angle θ of a reference line 500 of the left probe 110 belongs to among the first to third angle ranges 501 to 503.

That is, when the reference line 500 of the left probe 110 is located in the first angle range 501, the control unit 303 can determine that the relative angle between the pair of probes 100 is in the first state. In addition, when the reference line 500 of the left probe 110 is located in the second angular range 502, the control unit 303 can determine that the relative angle between the pair of probes 100 is in the second state. Finally, when the reference line 500 of the left probe 110 is located in the third angular range 503, the control unit 303 can determine that the relative angle between the pair of probes 100 is in the third state.

In an embodiment of the present invention, it is proposed to detect whether or not the user manipulates the buttons 210 and 220, and to block therapeutic light when it is determined that the user manipulates the buttons 210 and 220. This is because, in general, during treatment with therapeutic light, since the pair of probes 100 are fixed while being caught in the nasal septum 402, there is no need to manipulate the buttons 210 and 220, and when the user manipulates the buttons 210 and 220, the pair of probes 100 may move back and forth in and out of the nasal cavity as much as possible.

To this end, the sensing unit 304 may further detect manipulation of the buttons 210 and 220. For example, the sensing unit 304 includes a contact sensor for sensing the manipulation of the buttons 210 and 220 on the buttons 210 and 220, and when a user contacts the buttons 210 and 220 in order to manipulate the buttons 210 and 220, the contact sensor may sense the contact of the user and transmit the contact to the control unit 303.

As a sensor for manipulating the buttons 210 and 220, various types of sensors may be applied. For example, a proximity sensor that detects whether or not an object is in proximity, a pressure sensor that senses an applied pressure, and a switch-type sensor may be applied.

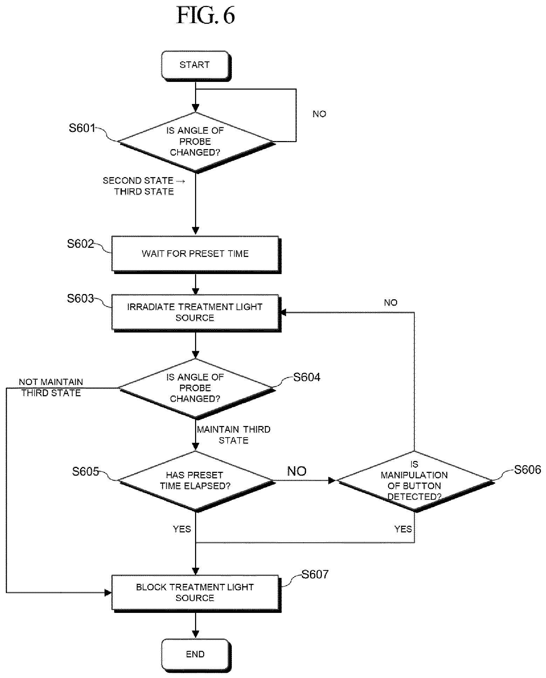

FIG. 6 is a view illustrating a control flow chart of the phototherapy apparatus A in accordance with still another exemplary embodiment of the present invention.

In step S601, the control unit 303 detects whether or not the angle of the pair of probes 100 changes through sensing by the sensing unit 304. In the case where the angle of the pair of probes 100 'changes from the second state to the third state', it proceeds to step S602, and in the case of not changing and in the remaining cases except for the case of 'changing from the second state to the third state', step S601 may be repeated.

In step S602, the control unit 303 may wait for a preset time. In this case, step S602 is not an essential step, and it may proceed directly to step S603 without step S602.

In step S603, the control unit 303 can control the light generation unit 302 to irradiate therapeutic light.

Subsequently, in step S604, the control unit 303 can additionally sense the angle of the pair of probes 100 through the sensing unit 304. In this case, the sensing of the angle may be whether or not the third state is maintained. If the third state is not maintained, the control unit 303 can proceed directly to step S607 and control to block therapeutic light.

This is because, if it is the case that the pair of probes 100 is being inserted continuously into the nasal cavity of the user, it is possible to determine that the angle of the pair of probes 100 will continue to maintain the third state.

For example, when the pair of probes 100 is slipped by an external shock or a runny nose while therapeutic light is being irradiated, the angle will change from the third state to the first state. In this case, since therapeutic light is to be blocked, the present invention proposes to maintain therapeutic light only when the third state is maintained.

If the third state is maintained in step S604, it proceeds to step S605. In step S605, it is determined whether or not a preset time for irradiating therapeutic light has elapsed. In this case, the preset time may be different from the preset time in step S602.

If the preset time has elapsed, it proceeds to step S607 to block therapeutic light. This is because the treatment is over. In this case, a guide notifying that the treatment has ended will be output to inform the user.

If the preset time has not yet elapsed, it proceeds to step S606 and detects whether or not the buttons 210 and 220 are manipulated. This is to immediately block therapeutic light when the user manipulates the buttons 210 and 220. Accordingly, it is possible to proceed to step S603 only when the manipulation of the buttons 210 and 220 is not detected, and repeat the process described above. When the manipulation of the buttons 210 and 220 is detected, the control unit 303 proceeds to step S607 to block therapeutic light.

The embodiments of the phototherapy apparatus and the control method thereof according to the present invention have been described above, but this is described as at least one embodiment, and the technical idea of the present invention and its configuration and operation are not limited thereto, and the scope of the technical idea of the present invention is not restricted/limited by the drawings or the description referring to the drawings. In addition, the concepts and embodiments of the invention presented in the present invention may be used by the person having ordinary skill in the art to which the present invention pertains as a basis for modifying or designing to other structures in order to perform the same object of the present invention, and the modified or altered equivalent structure by the person having ordinary skill in the art to which the present invention pertains is confined by the technical scope of the present invention set forth in the claims, and various changes, substitutions, and alterations are possible without departing from the spirit or scope of the invention set forth in the claims.

The effect of the phototherapy apparatus according to the present invention will be described as follows.

According to at least one of the embodiments of the present invention, there is an advantage that therapeutic light is irradiated only inside the nasal cavity and therapeutic light is blocked outside the nasal cavity, thereby improving safety.

Further scope of applicability of the present invention will become apparent from the detailed description below. However, since various alterations and modifications within the spirit and scope of the present invention can be clearly understood by the person skilled in the art, it should be understood that the detailed description and specific embodiments, such as preferred embodiments of the present invention, are given by way of example only.

Although the phototherapy apparatus preventing eye exposure and control method thereof have been described with reference to the specific embodiments, they are not limited thereto. Therefore, it will be readily understood by those skilled in the art that various modifications and changes can be made thereto without departing from the spirit and scope of the present invention defined by the appended claims.

## Claims

1. A phototherapy apparatus comprising:
a phototherapy apparatus body;
a pair of probes inserted into a nasal cavity;
a light generation unit configured to irradiate the nasal cavity with therapeutic light;
buttons configured to open tips of the pair of probes, at least one of the pair of probes being rotatably coupled to the phototherapy apparatus body;
a sensing unit configured to detect a manipulation of the button; and
a control unit configured to control the light generation unit based on a sensing result of the sensing unit.

2. The apparatus of claim 1, wherein the control unit is configured to control the light generation unit to block the therapeutic light when the manipulation is detected.

3. The apparatus of claim 1, further comprising:
an elastic portion configured to provide restoring force in a direction in which the tips of the pair of probes are closed.

4. The apparatus of claim 3, wherein the buttons are provided one by one on both sides of the phototherapy apparatus body, and the tips of the pair of probes are configured to be opened as much as the buttons are pressed on both sides of the phototherapy apparatus body.

5. The apparatus of claim 3, wherein the control unit is configured to control the light generation unit to block the therapeutic light when the tips of the pair of probes are closed by the restoring force.

6. The apparatus of claim 1, wherein the sensing unit is configured to include a contact sensor for detecting a user's finger, and to check whether or not the buttons are manipulated through whether a contact is made to the contact senor.

7. A control method of a phototherapy apparatus including a pair of probes inserted into a nasal cavity, the method comprising:
detecting a manipulation of buttons configured to open tips of a pair of probes; and
controlling a light generation unit for irradiating therapeutic light based on a detection result.

8. The method of claim 7, wherein, in the controlling the light generation unit, when the manipulation is detected, the light generation unit is controlled to block the therapeutic light.

9. The method of claim 7, wherein restoring force is provided in a direction in which the tips of the pair of probes are closed,
the buttons are provided one by one on both sides of the body, and
the tips of the pair of probes are opened as much as the buttons are pressed on both sides of a body of the phototherapy apparatus.

10. The method of claim 9, wherein, in the controlling the light generation unit, when the tips of the pair of probes are closed by the restoring force, the light generation unit controlled to block the therapeutic light.
